# EUROPEAN PATENT SPECIFICATION

(11) **EP 3 221 700 B1**
(45) Date of publication and mention of the grant of the patent: **22.06.2022**
(21) Application number: 15802296.2
(22) Date of filing: 17.11.2015
(51) Int. Cl.: G01N 33/574

(54) **PRKACB FUSIONS**
FUSIONEN VON PRKACB
FUSIONS DE PRKACB

(30) Priority: 18.11.2014 US 201462081445 P
(43) Date of publication of application: 27.09.2017
(73) Proprietor: Blueprint Medicines Corporation, Cambridge, MA 02139 (US)
(72) Inventor: STRANSKY, Nicolas, Charlestown, MA 02129 (US)
(74) Representative: Finnegan Europe LLP
(86) International application number: PCT/US2015/061073
(87) International publication number: WO 2016/081450

(56) References cited:
- WO-A1-2014/038884
- WO-A2-2004/073609
- HIROMI NAKAMURA ET AL: "Genomic spectra of biliary tract cancer", NATURE GENETICS., vol. 47, no. 9, 10 August 2015 (2015-08-10), pages 1003-1010, XP055250263, NEW YORK, US ISSN: 1061-4036, DOI: 10.1038/ng.3375

## Description

This invention relates to methods of detecting PRKACB (cAMP-dependent protein kinase catalytic subunit beta) gene fusions.

Many forms of cancer are caused by genetic lesions that give rise to tumor initiation and growth. Genetic lesions may include chromosomal aberrations, such as translocations, inversions, deletions, copy number changes, gene expression level changes, and somatic and germline mutations. Indeed, the presence of such genomic aberrations is a hallmark feature of many cancers, including, for example, B cell cancer, lung cancer, breast cancer, ovarian cancer, pancreatic cancer, and colon cancer. In some models, cancer represents the phenotypic end-point of multiple genetic lesions that endow cells with a full range of biological properties required for tumorigenesis.

Recent efforts by The Cancer Genome Atlas (TCGA), the International Cancer Genome Consortium (ICGC), and dozens of other large-scale profiling efforts have generated an enormous amount of new sequencing data for dozens of cancer types - this includes whole-genome DNA, whole-exome DNA, and full-transcriptome RNA sequencing. These efforts have led to the identification of new driver genes and fusion genes within multiple cancer types. Fusions, particularly fusions involving kinases, are of particular interest, as such fusions have been shown to be oncogenic, and have been successfully targeted by new therapeutics. For example, anaplastic lymphoma kinase (ALK), one of the receptor tyrosine kinases, is known to become oncogenic when fused with various genes. See, e.g., M. Soda et al, "Identification of the transforming EML4-ALK fusion gene in non-small-cell lung cancer," Nature 444:561-566 (2007).

A need exists for identifying novel genetic lesions associated with cancer. For example, the presence of fusions involving a kinase in samples collected from more than one source can indicate that the kinase is an oncogenic driver. The identification of such fusions can be an effective approach to diagnosis of cancers and development of compounds, compositions, methods, and assays for evaluating and treating cancer patients. WO 2014/038884 discloses a use of a fusion protein as a cancer diagnosis marker and/or a treatment target.

### SUMMARY

The invention is set out in the appended set of claims. In one aspect, the invention provides methods for detecting the presence of a PRKACB gene fusion in a biological sample, wherein the PRKACB gene fusion is a CEP170:PRKACB or RBM17:PRKACB fusion; the methods include the steps of: (a) contacting a biological sample from a mammal with a reagent that is an oligonucleotide that hybridizes to a fusion junction of the PRKACB gene fusion of SEQ ID NO: 1 or 3; and (b) detecting binding between the PRKACB gene fusion and the reagent. In some embodiments, the sample can be from, e.g., a cancer patient. In some embodiments, the cancer is breast cancer. In some embodiments, the cancer is head and neck squamous cell carcinoma.

### BRIEF DESCRIPTION OF THE FIGURES

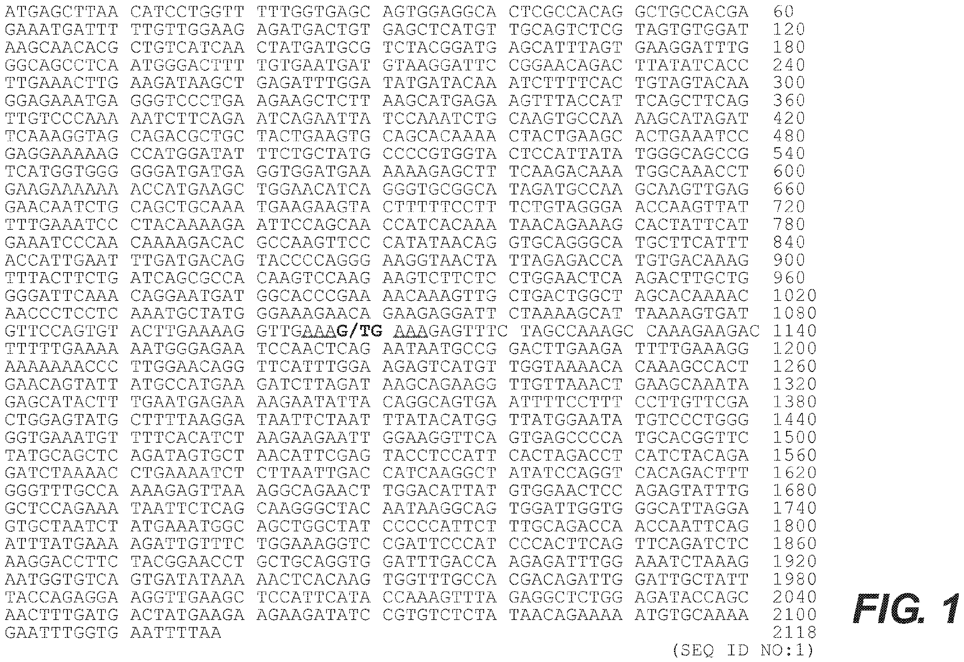

**FIG. 1** depict the nucleotide sequence of a CEP170:PRKACB gene fusion (SEQ ID NO:1) comprising exons 1-8 of the CEP170 gene (Accession No. NM_001042404) and exons 2-10 of the PRKACB gene (Accession No. NM_182948). The underlined codons at nucleotides 1105-1107 and 1111-1113 encode the last amino acid of CEP170 and the first amino acid of PRKACB, respectively. The slash after nucleotide 1108 indicates the breakpoint (fusion junction) where translocation and in-frame fusion has occurred. The shading at nucleotides 1108-1110 indicates that nucleotides from both CEP170 and PRKACB are fused in frame to form a codon and encode an amino acid.

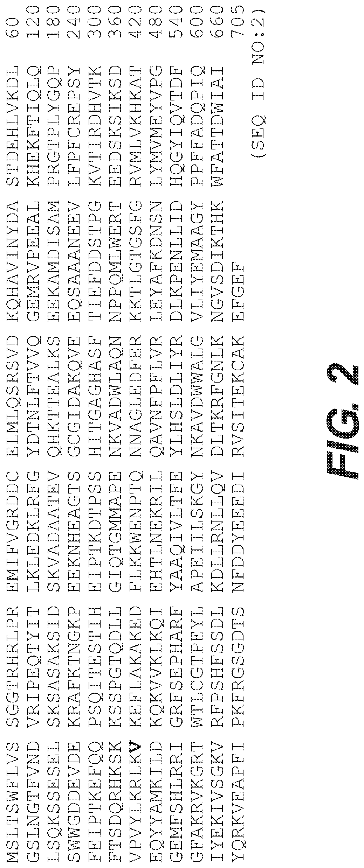

**FIG. 2** depicts the amino acid sequence of a CEP170:PRKACB fusion protein not according to the invention (SEQ ID NO:2). The shaded amino acid at position 370 corresponds to nucleotides 1108-1110 in SEQ ID NO:1. This amino acid is encoded by nucleotides from both CEP170 and PRKACB.

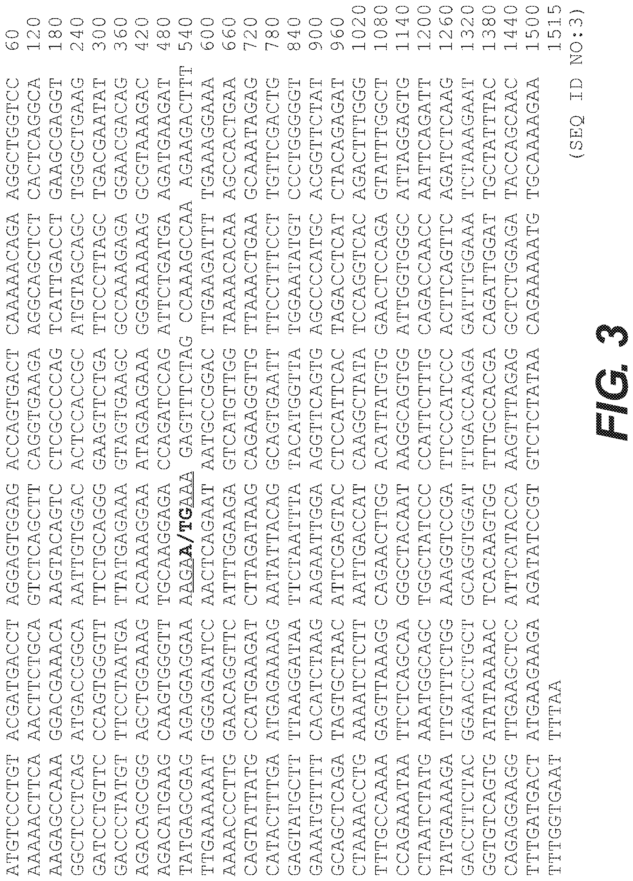

**FIG. 3** depicts the nucleotide sequence of an RBM17:PRKACB gene fusion (SEQ ID NO:2) comprising exons 1-5 of the RBM17 gene (Accession No. NM_032905) and exons 2-10 of the PRKACB gene (Accession No. NM_182948). The underlined codons at nucleotides 502-504 and 508-510 encode the last amino acid of RBM17 and the first amino acid of PRKACB, respectively. The slash after nucleotide 505 indicates the breakpoint (fusion junction) where translocation and in-frame fusion has occurred. The shading at nucleotides 505-507 indicates that nucleotides from both RBM17 and PRKACB are fused in frame to form a codon and encode an amino acid.

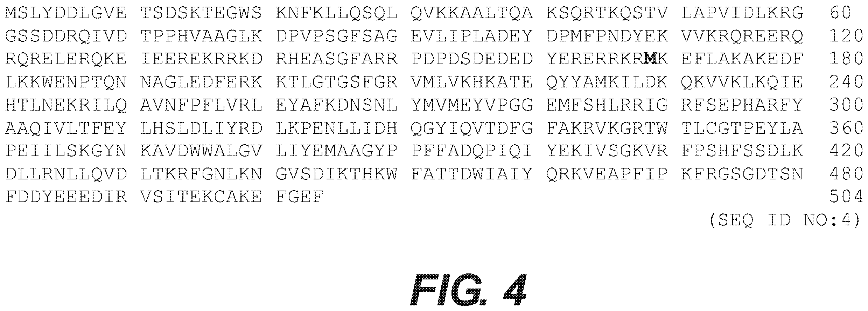

**FIG. 4** depicts the amino acid sequence of an RBM17:PRKACB fusion protein not according to the invention (SEQ ID NO:4). The shaded amino acid at position 169 corresponds to nucleotides 505-507 in SEQ ID NO:2. This amino acid is encoded by nucleotides from both RBM17 and PRKACB.

### EXEMPLARY EMBODIMENTS OF THE INVENTION

The invention is based, at least in part, on the discovery of novel recombination or translocation events in cancer patients that result in at least a fragment of a PRKACB gene linked to a non-homologous promoter via a recombination or translocation event that may result in aberrant expression and/or constitutive activation of PRKACB kinase activity. Thus, a new patient population is identified, which is characterized by the presence of a PRKACB fusion, e.g., a PRKACB gene fusion or fusion protein. This new patient population suffers from or is susceptible to disorders mediated by aberrant PRKACB expression or activity, or overexpression of PRKACB, such as, e.g., a cancer. In another aspect, a new subtype of cancer may be identified, which is characterized by the presence of the PRKACB fusions described herein. The new patient population may suffer from or may be susceptible to breast cancer or head and neck squamous cell carcinoma characterized by the presence of a PRKACB fusion. New methods of diagnosing and treating the patient population and the PRKACB fusion cancer subtype may be identified.

PRKACB is a member of the serine/threonine protein kinase family and is a catalytic subunit of protein kinase A (PKA, also known as cAMP-dependent protein kinase). PKA is activated by cyclic adenosine monophosphate (cAMP) and transduces the signal through phosphorylation of different target proteins. The inactive holoenzyme of PKA is a tetramer composed of two regulatory and two catalytic subunits. When two cAMP molecules bind to each PKA regulatory subunit, it causes the dissociation of the inactive holoenzyme into a dimer of regulatory subunits bound to four cAMP and two free monomeric catalytic subunits. The free catalytic subunits can then catalyze the transfer of ATP terminal phosphates to protein substrates at serine, or threonine residues. This phosphorylation can result in a change in activity of the substrate. For maximal function, each catalytic subunit of PKA can also be phosphorylated, which occurs on Thr 197 and helps orient catalytic residues in the active site. Since PKAs are present in a variety of cells and act on different substrates, PKA regulation is involved in many different pathways. For example, in direct protein phosphorylation, PKA directly either increases or decreases the activity of a protein. In protein synthesis, PKA first directly activates cAMP response element-binding protein (CREB), which binds the cAMP response element on a gene, altering the transcription of the gene and therefore the synthesis of the protein product.

The term "PRKACB fusion" is used generically herein, and includes any fusion molecule (e.g., gene, gene product (e.g., cDNA, mRNA, or protein), and variants thereof) that includes a fragment of PRKACB (in the case of a nucleotide sequence, including particularly the coding region for the kinase domain of PRKACB), and a fragment from a second non-homologous gene (in the case of a nucleotide sequence, including the promoter and/or the coding region of the non-homologous gene). A PRKACB fusion protein generally includes the kinase domain of PRKACB. The PRKACB fusion is a CEP170:PRKACB fusion, or an RBM17:PRKACB fusion. In some embodiments, the PRKACB fusions disclosed herein are associated with breast cancer. In some embodiments, the PRKACB fusions disclosed herein are associated with head and neck squamous cell carcinoma.

### PRKACB Gene Fusions and Fusion Proteins

PRKACB gene fusions are generated by a fusion between at least a part of the PRKACB gene and a part of another gene as a result of a translocation (including inversion) within a chromosome or between chromosomes. As a result of a translocation, the PRKACB gene may be placed under the transcriptional control of the partner gene promoter, resulting in aberrant expression or activity of PRKACB, or overexpression of PRKACB. As used herein, the 5'-region is upstream of, and the 3'-region is downstream of, a fusion junction or breakpoint in one of the component genes. PRKACB and the gene or protein that it is fused to is referred to as "fusion partners." Alternatively, they may be identified as a "PRKACB gene fusion" or a "PRKACB fusion protein" which are collectively termed "PRKACB fusions." The PRKACB fusions disclosed herein have a kinase activity. The phrase "having a kinase activity" as used in this application means having an activity as an enzyme phosphorylating the side chain of an amino acid, such as serine or threonine. In some embodiments, the PRKACB fusion may include an in-frame fusion of the coding sequences of PRKACB and the fusion partner that introduces amino acids into the fusion protein that are not part of PRKACB or the fusion partner.

In some exemplary embodiments, the fusion partner is all or a portion of CEP170 (centrosomal protein 170kDa). In other exemplary embodiments, the fusion partner is all or a portion of RBM17 (RNA binding motif protein 17, also known as splicing factor 45).

Reference to "all or a portion" or "all or part" of a PRKACB gene fusion or SEQ ID NO: 1 or 3, means that the nucleotide sequence comprises the entire PRKACB gene fusion nucleotide sequence or a fragment of that sequence that comprises the fusion junction or breakpoint between PRKACB and its fusion partner (CEP170 or RBM17). The fragment may comprise 7, 8, 9, 10, 12, 14, 16, 18, 20, 21, 22, 23, 24, 25, 26, 27, 28, 29, 30, 35, 40, 45, 50, 60, 70, 80, 90, 100, 120, 150, 175, 200, 250, 300, or more nucleotides spanning the fusion junction of the PRKACB gene fusion. Reference to "all or a portion" or "all or part" of a PRKACB fusion protein or SEQ ID NO:2 or 4, none of these being part of the invention, means an amino acid sequence that comprises the entire PRKACB fusion protein amino acid sequence or a fragment of that sequence that comprises the fusion junction or breakpoint between PRKACB and its fusion partner (CEP170 or RBM17). The fragment (not part of the invention) may comprise 8, 10, 12, 14, 15, 16, 18, 20, 21, 22, 23, 24, 25, 26, 27, 28, 29, 30, 35, 40, 45, 50, 75, or more amino acids spanning the fusion junction.

In one embodiment the invention is based on a fusion which includes an in-frame fusion of all or a portion of the CEP170 gene (e.g., a CEP170 promotor or a functional fragment thereof, and one or more exons encoding CEP170 or a fragment thereof) and an exon of the PRKACB gene (e.g., one or more exons encoding a PRKACB kinase domain or a functional fragment thereof). Such a fusion can be referred to as a CEP170:PRKACB fusion. The CEP170:PRKACB fusion may comprise sufficient PRKACB sequence to drive expression of a fusion protein that has kinase activity, e.g., has elevated activity as compared with wild type PRKACB in the same tissue or cell.

In a particular embodiment, the invention is based on a CEP170:PRKACB gene fusion comprising the nucleotide sequence depicted in Figure 1 (SEQ ID NO:1), or a fragment thereof that includes the fusion junction. SEQ ID NO:1 comprises CEP170 up to exon 8 fused to PRKACB, beginning at exon 2. The CEP170:PRKACB gene fusion comprises a nucleotide sequence that is at least 85%, at least 90%, at least 95%, at least 97%, at least 98%, or at least 99% identical to all or part of SEQ ID NO:1. In some embodiments, not part of the invention, the CEP170:PRKACB gene fusion encodes a protein having the sequence depicted in Figure 2 (SEQ ID NO:2) or a sequence that is at least 85%, at least 90%, at least 95%, at least 97%, at least 98%, or at least 99% identical to all or part of SEQ ID NO:2.

In one embodiment, the invention is based on a fusion including an in-frame fusion of all or a portion of the RBM17 gene (e.g., an RBM17 promotor or a functional fragment thereof, and one or more exons encoding RBM17 or a fragment thereof) and an exon of the PRKACB gene (e.g., one or more exons encoding a PRKACB kinase domain or a functional fragment thereof). Such a fusion can be referred to as an RBM17:PRKACB fusion. The RBM17:PRKACB fusion may comprise sufficient PRKACB sequence to drive expression of a fusion protein that has kinase activity, e.g., has elevated activity as compared with wild type PRKACB in the same tissue or cell.

In a particular embodiment, the invention is based on an RBM17:PRKACB gene fusion comprising the nucleotide sequence depicted in Figure 3 (SEQ ID NO:3), or a fragment thereof that includes the fusion junction. SEQ ID NO: 1 comprises RBM17 up to exon 5 fused to PRKACB, beginning at exon 2. The RBM17:PRKACB gene fusion comprises a nucleotide sequence that is at least 85%, at least 90%, at least 95%, at least 97%, at least 98%, or at least 99% identical to all or part of SEQ ID NO:3. The RBM17:PRKACB gene fusion encodes a protein having the sequence depicted in Figure 4 (SEQ ID NO:4) or a sequence that is at least 85%, at least 90%, at least 95%, at least 97%, at least 98%, at least 99% identical to all or part of SEQ ID NO:4.

The nucleic acid sequences of PRKACB gene fusions may be used as probes, primers, or bait to identify nucleotides from a biological sample that include, flank, or hybridize to PRKACB fusions, such as, e.g., CEP170:PRKACB (e.g., all or part of SEQ ID NO: 1), or RBM17:PRKACB (e.g., all or part of SEQ ID NO:3), at e.g., the fusion junctions. The probe, primer, or bait molecule is an oligonucleotide that allows capture, detection, and/or isolation of a PRKACB gene fusion in a biological sample. The probes or primers derived from the nucleic acid sequences of PRKACB gene fusions (e.g., from the fusion junctions) may be used, for example, for polymerase chain reaction (PCR) amplification. The oligonucleotide can comprise a nucleotide sequence substantially complementary to a fragment of the PRKACB gene fusion nucleic acid molecules described herein. The sequence identity between the nucleic acid fragment, e.g., the oligonucleotide and the target PRKACB gene fusion sequence, need not be exact, so long as the sequences are sufficiently complementary to allow the capture, detection, and/or isolation of the target sequence. In one embodiment, the nucleic acid fragment is a probe or primer that includes an oligonucleotide between about 5 and 25, e.g., between 10 and 20, or 10 and 15 nucleotides in length that includes the fusion junction of a PRKACB fusion of CEP170:PRKACB (e.g., all or part of SEQ ID NO: 1), or RBM17:PRKACB (e.g., all or part of SEQ ID NO:3). In other embodiments, the nucleic acid fragment is a bait that includes an oligonucleotide between about 100 to 300 nucleotides, 130 and 230 nucleotides, or 150 and 200 nucleotides in length that includes the fusion junction of a PRKACB fusion of CEP170:PRKACB (e.g., all or part of SEQ ID NO: 1), or RBM17:PRKACB (e.g., all or part of SEQ ID NO:3).

In certain embodiments, the nucleic acid fragments hybridize to a nucleotide sequence that includes a breakpoint or fusion junction, e.g., a breakpoint or fusion junction as identified by a slash ("/") in FIGs. 1 and 3. The nucleic acid fragment can hybridize to a nucleotide sequence that includes the fusion junction between the CEP170 transcript and the PRKACB transcript (e.g., nucleotides 1108-1110 of SEQ ID NO:1), or between the RBM17 transcript and the PRKACB transcript (e.g., nucleotides 505-507 of SEQ ID NO:3), i.e., a nucleotide sequence that includes a portion of SEQ ID NO: 1 or SEQ ID NO:3. Examples include a nucleotide sequence within exons 1-8 of a CEP170 gene and exons 2-10 of a PRKACB gene (e.g., a portion of SEQ ID NO:1 comprising nucleotides 1107-1111, 1104-1113, 1099-1118, 1084-1133, 1059-1158, 1034-1184, or 1009-1208); or a nucleotide sequence within exons 1-5 of an RBM17 gene and exons 2-10 of a PRKACB gene (e.g., a portion of SEQ ID NO:3 comprising nucleotides 504-508, 501-510, 496-515, 481-530, 456-555, 431-580, or 406-605).

In other embodiments, the nucleic acid fragment includes a bait that comprises a nucleotide sequence that hybridizes to a PRKACB gene fusion nucleic acid molecule described herein, and thereby allows the detection, capture, and/or isolation of the nucleic acid molecule. In one embodiment, a bait is suitable for solution phase hybridization. In other embodiments, a bait includes a binding entity or detection entity, e.g., an affinity tag or fluorescent label, that allows detection, capture, and/or separation, e.g., by binding to a binding entity, of a hybrid formed by a bait and a nucleic acid hybridized to the bait.

In exemplary embodiments, the nucleic acid fragments used as bait that includes a fusion junction between the CEP170 transcript and the PRKACB transcript, e.g., a nucleotide sequence within SEQ ID NO:1 comprising nucleotides 1108-1110 (such as, e.g., a sequence comprising nucleotides 1107-1111, 1104-1113, 1099-1118, 1084-1133, 1059-1158, 1034-1184, or 1009-1208 of SEQ ID NO:1).

In other exemplary embodiments, the nucleic acid sequences hybridize to a nucleotide sequence that includes a fusion junction between the RBM17 transcript and the PRKACB transcript, e.g., a nucleotide sequence within SEQ ID NO:3 comprising nucleotides 505-507 (such as, e.g., a sequence comprising nucleotides 504-508, 501-510, 496-515, 481-530, 456-555, 431-580, or 406-605 of SEQ ID NO:3)

Also disclosed but not according to the invention are PRKACB fusion proteins (such as, e.g., a purified or isolated CEP170:PRKACB, or RBM17:PRKACB fusion protein), biologically active or antigenic fragments thereof, and use of those polypeptides for detecting and/or modulating the biological activity (such as tumorigenic activity) of a PRKACB fusion protein. The PRKACB fusion proteins (not according to the invention) may comprise the amino acid sequence set forth in SEQ ID NO:2 or SEQ ID NO:4, and fragments of those sequences.

The PRKACB fusion protein (not according to the invention) can include a fragment of a CEP170 protein or an RBM17 protein, and a fragment of a PRKACB protein. The PRKACB fusion protein (not according to the invention) may be CEP170:PRKACB fusion protein having the amino acid sequence of SEQ ID NO:2 or a fragment thereof, such as, e.g., amino acids 368-372, 365-374, 360-379, 345-394, 320-419, 295-444, or 270-469 of SEQ ID NO:2. The PRKACB fusion protein (not according to the invention) may be an RBM17:PRKACB fusion protein having the amino acid sequence of SEQ ID NO:4 or a fragment thereof, such as, e.g., amino acids 167-171, 164-173, 159-178, 144-193, 119-218, 94-243, or 69-268 of SEQ ID NO:4.

The PRKACB fusion protein (not according to the invention) may be a CEP170:PRKACB fusion protein comprising an amino acid sequence that is at least 85%, at least 90%, at least 95%, at least 97%, at least 98%, or at least 99% identical to SEQ ID NO:2 or a fragment thereof (e.g., amino acids 368-372, 365-374, 360-379, 345-394, 320-419, 295-444, or 270-469 of SEQ ID NO:2). The PRKACB fusion protein (not according to the invention) may be an RBM17:PRKACB fusion protein comprising an amino acid sequence that is at least 85%, at least 90%, at least 95%, at least 97%, at least 98%, or at least 99% identical to SEQ ID NO:4 or a fragment thereof (e.g., amino acids 167-171, 164-173, 159-178, 144-193, 119-218, 94-243, or 69-268).

The PRKACB fusion protein (not according to the invention) may include a functional kinase domain. The PRKACB fusion protein (not according to the invention) may comprise elevated PRKACB activity as compared with wild type PRKACB activity (e.g., in a cancer cell, a non-cancer cell adjacent to the cancer cell, or a non-cancer cell from a control sample, such as a cancer free subject). The PRKACB fusion protein (not according to the invention) may be a CEP170:PRKACB fusion and includes a PRKACB serine/threonine kinase domain or a functional fragment thereof. The PRKACB fusion protein (not according to the invention) may be an RBM17:PRKACB fusion and includes a PRKACB serine/threonine kinase domain or a functional fragment thereof.

The PRKACB fusion protein or fragment (not according to the invention) may be a peptide, e.g., an immunogenic peptide or protein, that contains a fusion junction with a heterologous protein as described herein. Such immunogenic peptides or proteins can be used for vaccine preparation for use in the treatment or prevention of cancers caused by or exacerbated by PRKACB gene fusions and PRKACB fusion proteins. Such immunogenic peptides or proteins can be used to raise antibodies specific to the fusion protein. The PRKACB fusion protein (not according to the invention) may be present in combination with or is further conjugated to one or more adjuvant(s) or immunogen(s), e.g., a protein capable of enhancing an immune response to the PRKACB fusion protein (e.g., a hapten, a toxoid, etc.). The PRKACB fusion protein (not according to the invention) may be a CEP170: PRKACB or RBM17: PRKACB fusion protein. The PRKACB fusion protein (not according to the invention) may comprise the fusion junction of SEQ ID NO:2 or SEQ ID NO:4.

Also disclosed but not according to the invention is an antibody that binds to a PRKACB fusion protein (such as, e.g., a CEP170:PRKACB or an RBM17:PRKACB fusion protein) or a fragment thereof. The antibody (not according to the invention) may recognize a PRKACB fusion protein but does not recognize wild type PRKACB or the wild type fusion partner (such as, e.g., CEP170 or RBM17). The antibody (not according to the invention) may bind to an epitope comprising the fusion junction between PRKACB and the fusion partner (e.g., the junction of CEP170:PRKACB or RBM17:PRKACB). The antibody (not according to the invention) may bind to a CEP170:PRKACB fusion protein having the amino acid sequence of SEQ ID NO:2 or a fragment thereof, such as, e.g., amino acids 368-372, 365-374, 360-379, 345-394, 320-419, 295-444, or 270-469 of SEQ ID NO:2. The antibody (not according to the invention) may bind to an RBM17:PRKACB fusion protein having the amino acid sequence of SEQ ID NO:4 or a fragment thereof, such as, e.g., amino acids 167-171, 164-173, 159-178, 144-193, 119-218, 94-243, or 69-268 of SEQ ID NO:4.

The antibodies (not according to the invention) may inhibit and/or neutralize the biological activity of the PRKACB fusion protein, and more specifically, in some embodiments, the kinase activity of the PRKACB fusion protein. The antibodies (not according to the invention) may be used to detect a PRKACB fusion protein or to diagnose a patient suffering from a disease or disorder associated with the expression of a PRKACB fusion protein.

### Detection and Diagnostic Methods

In another aspect, the invention provides a method of determining the presence of a PRKACB gene fusion that is a CEP170:PRKACB or an RBM17:PRKACB fusion as described herein. The presence of a PRKACB gene fusion can indicate that the mammal providing the biological sample suffers from or is at risk of developing a disorder mediated by aberrant PRKACB expression or activity, or overexpression of PRKACB, such as, e.g., a cancer. The presence of a PRKACB gene fusion may also indicate that the cancer is treatable with a PRKACB inhibitor (such as, e.g., a kinase inhibitor or an antibody specific to PRKACB) or a PRKACB fusion inhibitor. In some embodiments, the cancer is breast cancer. In some embodiments, the cancer is head and neck squamous cell carcinoma. In some embodiments, the PRKACB fusion present in the sample is CEP170:PRKACB and the cancer to be treated is breast cancer. In other embodiments, the PRKACB fusion present in the sample is RBM17:PRKACB and the cancer to be treated is head and neck squamous cell carcinoma. In other embodiments, the cancer is a different cancer associated with aberrant expression or activity of PRKACB or overexpression of PRKACB.

The PRKACB fusion detected is a nucleic acid molecule. The method includes detecting whether a PRKACB fusion nucleic acid molecule is present in a cell (e.g., a circulating cell or a cancer cell), a tissue (e.g., a tumor), or a sample, e.g., a tumor sample, from a subject. In one embodiment, the sample is a nucleic acid sample. In one embodiment, the nucleic acid sample comprises DNA, e.g., genomic DNA or cDNA, or RNA, e.g., mRNA.

The sample can be chosen from one or more sample types, such as, for example, tissue, e.g., cancerous tissue (e.g., a tissue biopsy), whole blood, serum, plasma, buccal scrape, sputum, saliva, cerebrospinal fluid, urine, stool, circulating tumor cells, circulating nucleic acids, or bone marrow.

### I. Methods for Detecting Gene Fusions

In some embodiments, the PRKACB fusion is detected in a nucleic acid molecule by one or more methods chosen from nucleic acid hybridization assays (e.g. in situ hybridization, comparative genomic hybridization, microarray, Southern blot, northern blot), amplification-based assays (e.g., PCR, PCR-RFLP assay, or real-time PCR), sequencing and genotyping (e.g. sequence-specific primers, high-performance liquid chromatography, or mass-spectrometric geno-typing), and screening analysis (including metaphase cytogenetic analysis by karyotype methods).

### (1) Hybridization methods

In some embodiments, the reagent hybridizes to a PRKACB gene fusion, such as, e.g., nucleotides 1108-1110, 1107-1111, 1104-1113, 1099-1118, 1084-1133, 1059-1158, 1034-1184, or 1009-1208 of SEQ ID NO:1. In alternate embodiments, the reagent detects the presence of nucleotides 505-507, 504-508, 501-510, 496-515, 481-530, 456-555, 431-580, or 406-605 of SEQ ID NO:3. In an alternate embodiment, the method includes the steps of obtaining a sample; exposing the sample to a nucleic acid probe which hybridizes to an mRNA or cDNA encoding a PRKACB fusion protein that comprises amino acids 368-372, 365-374, 360-379, 345-394, 320-419, 295-444, or 270-469 of SEQ ID NO:2, or amino acids 167-171, 164-173, 159-178, 144-193, 119-218, 94-243, or 69-268 of SEQ ID NO:4.

Hybridization, as described throughout the specification, may be carried out under stringent conditions, e.g., medium or high stringency. See, e.g., J. Sambrook, E.F. Fritsch, and T. Maniatis, Molecular Cloning: A Laboratory Manual, Cold Spring Harbor Laboratory Pr; 2nd edition (1989); T. Brown, Hybridization Analysis of DNA Blots. Current Protocols in Molecular Biology at 21:2.10.1-2.10.16 (2001). High stringency conditions for hybridization refer to conditions under which two nucleic acids must possess a high degree of base pair homology to each other in order to hybridize. Examples of highly stringent conditions for hybridization include hybridization in 4×sodium chloride/sodium citrate (SSC), at 65 or 70° C., or hybridization in 4×SSC plus 50% formamide at about 42 or 50° C., followed by at least one, at least two, or at least three washes in 1 × SSC, at 65 or 70° C. Another example of highly stringent conditions includes hybridization in 2×SSC; 10×Denhardt solution (Fikoll 400+PEG+BSA;ratio 1:1:1); 0.1% SDS; 5 mM EDTA; 50 mM Na₂HPO₄;250 µg/ml of herring sperm DNA; 50 µg/ml of tRNA; or0.25 M of sodium phosphate buffer, pH 7.2;1 mM EDTA7% SDS at 60° C; followed by washing 2×SSC, 0.1% SDS at 60° C.

The nucleic acid fragments can be detectably labeled with, e.g., a radiolabel, a fluorescent label, a bioluminescent label, a chemiluminescent label, an enzyme label, a binding pair label (e.g., biotin/streptavidin), an antigen label, or can include an affinity tag, or identifier (e.g., an adaptor, barcode or other sequence identifier). Labeled or unlabeled nucleic acids and/or nucleic acid fragments may be used in reagents for detecting, capturing, and/or isolating PRKACB gene fusions that are CEP170:PRKACB (e.g., all or part of SEQ ID NO: 1), or RBM17:PRKACB (e.g., all or part of SEQ ID NO:3). In some embodiments, the labeled reagent can be detected using, e.g., autoradiography, microscopy (e.g., brightfield, fluorescence, or electron microscopy), enzyme-linked immunosorbent assay (ELISA), or immunohistochemistry.

In one embodiment, the method includes: contacting a nucleic acid sample, e.g., a genomic DNA sample (e.g., a chromosomal sample or a fractionated, enriched or otherwise pretreated sample) or a gene product (mRNA, or cDNA), obtained from the subject, with a nucleic acid fragment e.g., a probe or primer as described herein (e.g., an exon-specific or a breakpoint-specific probe or primer) under conditions suitable for hybridization, and determining the presence or absence of the PRKACB gene fusion that is CEP170:PRKACB or RBM17:PRKACB, as disclosed herein.

In some embodiments, the method comprises performing chromosome in situ hybridization with chromosomal DNA from a biological sample to detect the presence of a PRKACB gene fusion (CEP170:PRKACB or RBM17:PRKACB, as disclosed herein). In some embodiments, the chromosome in situ hybridization comprises the steps of: providing a chromosome (e.g., interphase or metaphase chromosome) preparation (e.g., by attaching the chromosomes to a substrate (e.g., glass)); denaturing the chromosomal DNA (e.g., by exposure to formamide) to separate the double strands of the polynucleotides from each other; exposing the nucleic acid probe to the chromosomes under conditions to allow hybridization of the probe to the target DNA; removing unhybridized or non-specifically hybridized probes by washing; and detecting the hybridization of the probe with the target DNA. In some embodiments, the chromosome in situ hybridization is fluorescence in situ hybridization (FISH). In some embodiments, the probe is labeled directly by a fluorescent label, or indirectly by incorporation of a nucleotide containing a tag or reporter molecule (e.g., biotin, digoxigenin, or hapten) which after hybridization to the target DNA is then bound by fluorescently labeled affinity molecule (e.g., an antibody or streptavidin). In some embodiments, the hybridization of the probe with the target DNA in FISH can be visualized using a fluorescence microscope.

In other embodiments, the method comprises performing Southern blot with DNA polynucleotides from a biological sample to detect the presence of a PRKACB gene fusion (CEP170:PRKACB or RBM17:PRKACB, as disclosed herein). In some embodiments, the Southern blot comprises the steps of: optionally fragmenting the polynucleotides into smaller sizes by restriction endonucleases; separating the polynucleotides by gel electrophoresis; denaturing the polynucleotides (e.g., by heat or alkali treatment) to separate the double strands of the polynucleotides from each other; transferring the polynucleotides from the gel to a membrane (e.g., a nylon or nitrocellulose membrane); immobilizing the polynucleotides to the membrane (e.g., by UV light or heat); exposing the nucleic acid probe to the polynucleotides under conditions to allow hybridization of the probe to the target DNA; removing unhybridized or non-specifically hybridized probes by washing; and detecting the hybridization of the probe with the target DNA.

### (2) Amplification-based assays

In certain embodiments, the method of determining the presence of a PRKACB gene fusion, comprises (a) performing a PCR amplification reaction with polynucleotides from a biological sample, wherein the amplification reaction utilizes a pair of primers which will amplify at least a fragment of the PRKACB gene fusion, wherein the fragment comprises the fusion junction, wherein the first primer is in sense orientation and the second primer is in antisense orientation; and (b) detecting an amplification product, wherein the presence of the amplification product is indicative of the presence of a PRKACB fusion polynucleotide in the sample. In specific exemplary embodiments, the PRKACB gene fusion is CEP170:PRKACB, such as, e.g., the gene fusion of SEQ ID NO: 1 or a fragment thereof comprising nucleotides 1108-1110, 1107-1111, 1104-1113, 1099-1118, 1084-1133, 1059-1158, 1034-1184, or 1009-1208 of SEQ ID NO:1. In other exemplary embodiments, the gene fusion is RBM17:PRKACB such as, e.g. the gene fusion of SEQ ID NO:3 or a fragment thereof comprising nucleotides 505-507, 504-508, 501-510, 496-515, 481-530, 456-555, 431-580, or 406-605 of SEQ ID NO:3.

In some embodiments, step (a) of performing a PCR amplification reaction comprises: (i) providing a reaction mixture comprising the polynucleotides (e.g., DNA or cDNA) from the biological sample, the pair of primers which will amplify at least a fragment of the PRKACB gene fusion wherein the first primer is complementary to a sequence on the first strand of the polynucleotides and the second primer is complementary to a sequence on the second strand of the polynucleotides, a DNA polymerase, and a plurality of free nucleotides comprising adenine, thymine, cytosine, and guanine (dNTPs); (ii) heating the reaction mixture to a first predetermined temperature for a first predetermined time to separate the double strands of the polynucleotides from each other; (iii) cooling the reaction mixture to a second predetermined temperature for a second predetermined time under conditions to allow the first and second primers to hybridize with their complementary sequences on the first and second strands of the polynucleotides, and to allow the DNA polymerase to extend the primers; and (iv) repeating steps (ii) and (iii) for a predetermined number of cycles (e.g., 10, 15, 20, 25, 30, 35, 40, 45, or 50 cycles).

In some embodiments, the polynucleotides from the biological sample comprise RNA, and the method further comprises performing a RT-PCR amplification reaction with the RNA to synthesize cDNA as the template for subsequent or simultaneous PCR reactions. In some embodiments, the RT-PCR amplification reaction comprises providing a reaction mixture comprising the RNA, a primer which will amplify the RNA (e.g., a sequence-specific primer, a random primer, or oligo(dT)s), a reverse transcriptase, and dNTPs, and heating the reaction mixture to a third predetermined temperature for a third predetermined time under conditions to allow the reverse transcriptase to extend the primer.

### (3) Sequencing and Genotyping (not according to the invention)

Another method for determining the presence of a PRKACB gene fusion molecule (such as, e.g., CEP170:PRKACB or RBM17:PRKACB, as disclosed herein) includes: sequencing a portion of the nucleic acid molecule (e.g., sequencing the portion of the nucleic acid molecule that comprises the fusion junction of a PRKACB gene fusion), thereby determining that the PRKACB gene fusion is present in the nucleic acid molecule. Optionally, the sequence acquired is compared to a reference sequence, or a wild type reference sequence. The sequence may be determined by a next generation sequencing method. The sequencing may be automated and/or high-throughput sequencing. The method can further include acquiring, e.g., directly or indirectly acquiring, a sample, e.g., a tumor or cancer sample, from a patient.

The sequencing may comprise chain terminator sequencing (Sanger sequencing), comprising: providing a reaction mixture comprising a nucleic acid molecule from a biological sample, a primer complementary to a region of the template nucleic acid molecule, a DNA polymerase, a plurality of free nucleotides comprising adenine, thymine, cytosine, and guanine (dNTPs), and at least one chain terminating nucleotide (e.g., at least one di-deoxynucleotide (ddNTPs) chosen from ddATP, ddTTP, ddCTP, and ddGTP), wherein the at least one chain terminating nucleotide is present in a low concentration so that chain termination occurs randomly at any one of the positions containing the corresponding base on the DNA strand; annealing the primer to a single strand of the nucleic acid molecule; extending the primer to allow incorporation of the chain terminating nucleotide by the DNA polymerase to produce a series of DNA fragments that are terminated at positions where that particular nucleotide is used; separating the polynucleotides by electrophoresis (e.g., gel or capillary electrophoresis); and determining the nucleotide order of the template nucleic acid molecule based on the positions of chain termination on the DNA fragments. The sequencing may be carried out with four separate base-specific reactions, wherein the primer or the chain terminating nucleotide in each reaction is labeled with a separate fluorescent label. The sequencing may be carried out in a single reaction, wherein the four chain terminating nucleotides mixed in the single reaction are each labeled with a separate fluorescent label.

The sequencing may comprise pyrosequencing (sequencing by synthesis), comprising: (i) providing a reaction mixture comprising a nucleic acid molecule from a biological sample, a primer complementary to a region of the template nucleic acid molecule, a DNA polymerase, a first enzyme capable of converting pyrophosphate into ATP, and a second enzyme capable using ATP to generates a detectable signal (e.g., a chemiluminescent signal, such as light) in an amount that is proportional to the amount of ATP; (ii) annealing the primer to a single strand of the nucleic acid molecule; (iii) adding one of the four free nucleotides (dNTPs) to allow incorporation of the correct, complementary dNTP onto the template by the DNA polymerase and release of pyrophosphate stoichiometrically; (iv) converting the released pyrophosphate to ATP by the first enzyme; (v) generating a detectable signal by the second enzyme using the ATP; (vi) detecting the generated signal and analyzing the amount of signal generated in a pyrogram; (vii) removing the unincorporated nucleotides; and (viii) repeating steps (iii) to (vii). The method allows sequencing of a single strand of DNA, one base pair at a time, and detecting which base was actually added at each step. The solutions of each type of nucleotides are sequentially added and removed from the reaction. Light is produced only when the nucleotide solution complements the first unpaired base of the template. The order of solutions which produce detectable signals allows the determination of the sequence of the template.

The method of determining the presence of a PRKACB fusion (such as, e.g., CEP170:PRKACB or RBM17:PRKACB, as disclosed herein) may comprise analyzing a nucleic acid sample (e.g., DNA, cDNA, or RNA, or an amplification product thereof) by HPLC. The method may comprise: passing a pressurized liquid solution containing the sample through a column filled with a sorbent, wherein the nucleic acid or protein components in the sample interact differently with the sorbent, causing different flow rates for the different components; separating the components as they flow out the column at different flow rates. The HPLC may be chosen from, e.g., reverse-phase HPLC, size exclusion HPLC, ion-exchange HPLC, and bioaffinity HPLC.

The method of determining the presence of a PRKACB fusion (such as, e.g., CEP170:PRKACB or RBM17:PRKACB, as disclosed herein) may comprise analyzing a nucleic acid sample (e.g., DNA, cDNA, or RNA, or an amplification product thereof) by mass spectrometry. The method may comprise: ionizing the components in the sample (e.g., by chemical or electron ionization); accelerating and subjecting the ionized components to an electric or magnetic field; separating the ionized components based on their mass-to-charge ratios; and detecting the separated components by a detector capable of detecting charged particles (e.g., by an electron multiplier).

### II. Methods for detecting Fusion Proteins (not according to the invention)

Also disclosed is a method of determining the presence of a PRKACB fusion protein (such as, e.g., CEP170:PRKACB or RBM17:PRKACB, as disclosed herein) in a mammal. The method comprises the steps of obtaining a biological sample of a mammal (such as, e.g., a human cancer), and exposing that sample to at least one reagent that detects a PRKACB fusion protein (e.g., an antibody that recognizes the PRKACB fusion but does not recognize the wild type PRKACB or the wild type fusion partner) to determine whether a PRKACB fusion protein is present in the biological sample. The detection of a PRKACB fusion protein indicates the presence of a mutant PRKACB in the mammal (such as, e.g, in the human cancer). The PRKACB fusion protein may comprise an amino acid sequence having at least 85%, 90%, 95%, 97%, 98%, or 99% identity with an amino acid sequence of all or part of SEQ ID NO: 2 or SEQ ID NO:4. The cancer may be breast cancer. The cancer may ber head and neck squamous cell carcinoma.

The reagent that detects a PRKACB fusion protein can be detectably labeled with, e.g., a radiolabel, a fluorescent label, a bioluminescent label, a chemiluminescent label, an enzyme label, a binding pair label (e.g., biotin/streptavidin), an antigen label, or can include an affinity tag or identifier (e.g., an adaptor, barcode or other sequence identifier). The labeled reagent can be detected using, e.g., autoradiography, microscopy (e.g., brightfield, fluorescence, or electron microscopy), ELISA, or immunohistochemistry. The PRKACB fusion protein may be detected in a biological sample by a method chosen from one or more of: antibody-based detection (e.g., western blot, ELISA, immunohistochemistry), size-based detection methods (e.g., HPLC or mass spectrometry), or protein sequencing.

### (1) Antibody-based detection (not according to the invention)

The method may comprise performing a western blot with polypeptides from a biological sample to detect the presence of a PRKACB fusion protein (such as, e.g., CEP170:PRKACB or RBM17:PRKACB, as disclosed herein). The western blot may comprise the steps of: separating the polypeptides by gel electrophoresis; transferring the polypeptides from the gel to a membrane (e.g., a nitrocellulose or polyvinylidene difluoride (PVDF) membrane); blocking the membrane to prevent nonspecific binding by incubating the membrane in a dilute solution of protein (e.g., 3-5% bovine serum albumin (BSA) or non-fat dry milk in Tris-Buffered Saline (TBS) or I-Block, with a minute percentage (e.g., 0.1%) of detergent, such as, e.g., Tween 20 or Triton X-100); exposing the polypeptides to at least one reagent that detects a PRKACB fusion protein (e.g., an antibody that recognizes the PRKACB fusion but does not recognize the wild type PRKACB or the wild type fusion partner); removing unbound or non-specifically bound reagent by washing; and detecting the binding of the reagent with the target protein. The method may comprise two-step detection: exposing the polypeptides to a primary antibody that specifically binds to a PRKACB fusion protein; removing unbound or non-specifically bound primary antibody by washing; exposing the polypeptides to a secondary antibody that recognizes the primary antibody; removing unbound or non-specifically bound secondary antibody by washing; and detecting the binding of the secondary antibody. The reagent that detects a PRKACB fusion protein (e.g., the fusion specific antibody, or the secondary antibody) may be directly labeled for detection. The reagent may be linked to an enzyme, and the method may further comprise adding a substrate of the enzyme to the membrane; and developing the membrane by detecting a detectable signal produced by the reaction between the enzyme and the substrate. For example, the reagent may be linked with horseradish peroxidase to cleave a chemiluminescent agent as a substrate, producing luminescence in proportion to the amount of the target protein for detection.

The method may comprise performing ELISA with polypeptides from a biological sample to detect the presence of a PRKACB fusion protein (such as, e.g., CEP170:PRKACB, or RBM17:PRKACB, as disclosed herein). The ELISA may be chosen from, e.g., direct ELISA, indirect ELISA, sandwich ELISA, and competitive ELISA.

The direct ELISA may comprise the steps of: attaching polypeptides from a biological sample to a surface; blocking the surface to prevent nonspecific binding by incubating the surface in a dilute solution of protein; exposing the polypeptides to an antibody that specifically binds to a PRKACB fusion protein (e.g., an antibody that recognizes the PRKACB fusion (such as, e.g., CEP170:PRKACB, or RBM17:PRKACB, as disclosed herein) but does not recognize the wild type PRKACB or the wild type fusion partner); removing unbound or non-specifically bound antibody by washing; and detecting the binding of the antibody with the target protein. The antibody may be directly labeled for detection. The antibody may be linked to an enzyme, and the method may further comprise adding a substrate of the enzyme; and detecting a detectable signal produced by the reaction between the enzyme and the substrate.

The indirect ELISA may comprise the steps of: attaching polypeptides from a biological sample to a surface; blocking the surface to prevent nonspecific binding by incubating the surface in a dilute solution of protein; exposing the polypeptides to a primary antibody that specifically binds to a PRKACB fusion protein (such as, e.g., CEP170:PRKACB, or RBM17:PRKACB, as disclosed herein); removing unbound or non-specifically bound primary antibody by washing; exposing the polypeptides to a secondary antibody that recognizes the primary antibody; removing unbound or non-specifically bound secondary antibody by washing; and detecting the binding of the secondary antibody. The secondary antibody may be directly labeled for detection. The secondary antibody may be linked to an enzyme, and the method may further comprise adding a substrate of the enzyme; and detecting a detectable signal produced by the reaction between the enzyme and the substrate.

The method may comprise performing immunohistochemistry with polypeptides from a biological sample to detect the presence of a PRKACB fusion protein (such as, e.g., CEP170:PRKACB or RBM17:PRKACB, as disclosed herein). The immunohistochemistry may comprise the steps of: fixing a cell or a tissue section (e.g., by paraformaldehyde or formalin treatment); permeabilizing the cell or tissue section to allow target accessibility; blocking the cell or tissue section to prevent nonspecific binding; exposing the cell or tissue section to at least one reagent that detects a PRKACB fusion protein (e.g., an antibody that recognizes the PRKACB fusion but does not recognize the wild type PRKACB or the wild type fusion partner); removing unbound or non-specifically bound reagent by washing; and detecting the binding of the reagent with the target protein. The reagent may be directly labeled for detection. The reagent may be linked to an enzyme, and the method may further comprise adding a substrate of the enzyme; and detecting a detectable signal produced by the reaction between the enzyme and the substrate. The immunohistochemistry may comprise the two-step detection as in the indirect ELISA.

### Size-based detection methods (not according to the invention)

The method of determining the presence of a PRKACB fusion (such as, e.g., CEP170:PRKACB or RBM17:PRKACB, as disclosed herein) may comprise analyzing a protein sample by HPLC. The method may comprise: passing a pressurized liquid solution containing the sample through a column filled with a sorbent, wherein the nucleic acid or protein components in the sample interact differently with the sorbent, causing different flow rates for the different components; separating the components as they flow out the column at different flow rates. The HPLC may be chosen from, e.g., reverse-phase HPLC, size exclusion HPLC, ion-exchange HPLC, and bioaffinity HPLC.

The method of determining the presence of a PRKACB fusion (such as, e.g., CEP170:PRKACB or RBM17:PRKACB, as disclosed herein) may comprise analyzing a protein sample by mass spectrometry. The method may comprise: ionizing the components in the sample (e.g., by chemical or electron ionization); accelerating and subjecting the ionized components to an electric or magnetic field; separating the ionized components based on their mass-to-charge ratios; and detecting the separated components by a detector capable of detecting charged particles (e.g., by an electron multiplier).

Detection of a PRKACB gene fusion or a PRKACB fusion protein in a patient can lead to assignment of the patient to the newly identified patient population that bears the PRKACB fusion. Because this patient population can suffer from or be susceptible to a disorder associated with an aberrant PRKACB expression or activity or overexpression of PRKACB, detection of the PRKACB fusion can also lead to diagnosis of such disorder. Also disclosed is a method of stratifying a patient population (e.g., assigning a patient, to a group or class) and/or diagnosing a patient, comprising: obtaining a biological sample from the patient, contacting the sample with at least one reagent that detects a PRKACB gene fusion or a PRKACB fusion protein to determine whether a PRKACB fusion is present in the biological sample. The detection of a PRKACB fusion indicates that the patient belongs to the newly identified patient population that bears the PRKACB fusion, and/or the presence of a disorder associated with aberrant PRKACB expression or activity or overexpression or PRKACB, such as e.g., a cancer. The detection of a PRKACB fusion also identifies a new subtype of cancer, which is characterized by the presence of the PRKACB fusion. The cancer may be breast cancer. The cancer may be head and neck squamous cell carcinoma. The PRKACB fusion may be CEP170:PRKACB. The PRKACB fusion may be RBM17:PRKACB. The CEP170:PRKACB fusion may have all or a part of the nucleotide and/or amino acid sequence (such as, e.g., the fusion junction) set forth in SEQ ID NO:1 and SEQ ID NO:2, respectively. The RBM17:PRKACB fusion may have all or part of the nucleotide and/or amino acid sequence (such as, e.g., the fusion junction) set forth in SEQ ID NO:3 and SEQ ID NO:4, respectively.

The PRKACB gene fusion or PRKACB fusion protein may be detected prior to initiating, during, and/or after, a treatment of a patient with, e.g., a PRKACB inhibitor or a PRKACB fusion inhibitor. The PRKACB gene fusion or PRKACB fusion protein may be detected at the time the patient is diagnosed with a cancer. The PRKACB fusion may be detected at a pre-determined interval, e.g., a first point in time and at least at a subsequent point in time. In response to detection of a PRKACB fusion, such as, e.g., CEP170:PRKACB or RBM17:PRKACB, the method may further include one or more of:
(1) stratifying a patient population (e.g., assigning a patient, to a group or class);
(2) identifying or selecting the patient as likely or unlikely to respond to a treatment, e.g., a PRKACB inhibitor treatment (e.g., a kinase inhibitor treatment), or a PRKACB fusion inhibitor treatment as described herein;
(3) selecting a treatment regimen, e.g., administering or not administering a preselected therapeutic agent, such as, e.g., a PRKACB inhibitor, or a PRKACB fusion inhibitor;
(4) prognosticating the time course of the disease in the patient (e.g., evaluating the likelihood of increased or decreased patient survival); or
(5) monitoring the effectiveness of treatment (e.g., by detecting a reduction in the level of PRKACB gene fusion or fusion protein in a patient sample).

Upon detection of a PRKACB gene fusion or PRKACB fusion protein in a patient's biological sample, the patient may be identified as likely to respond to a treatment that comprises a PRKACB inhibitor, or a PRKACB fusion inhibitor. The PRKACB fusion detected may be a CEP170:PRKACB fusion. The PRKACB fusion detected may be an RBM17:PRKACB fusion.

Also disclosed is a method of selecting a treatment option by detecting a PRKACB fusion. The method comprises obtaining a biological sample from a patient and exposing the sample to at least one reagent that detects a PRKACB gene fusion or fusion protein to determine whether a PRKACB fusion is present in the biological sample. The detection of the PRKACB fusion indicates the likelihood of the patient responding to treatment with a PRKACB inhibitor, or a PRKACB fusion inhibitor. The method may be augmented or personalized by evaluating the effect of a variety of PRKACB or PRKACB fusion inhibitors on the biological sample shown to contain a PRKACB fusion to determine the most appropriate inhibitor to administer. The PRKACB fusion may be CEP170:PRKACB. The PRKACB fusion may be RBM17:PRKACB. The CEP170:PRKACB fusion may have all or a part of the nucleotide and/or amino acid sequence (such as, e.g., the fusion junction) set forth in SEQ ID NO:1 and SEQ ID NO:2, respectively. The RBM17:PRKACB fusion may have all or part of the nucleotide and/or amino acid sequence (such as, e.g., the fusion junction) set forth in SEQ ID NO:3 and SEQ ID NO:4, respectively.

### Methods of Treatment (not according to the invention)

Alternatively, or in combination with the detection and diagnostic methods described herein, methods are disclosed for treating the newly identified patient population and the new PRKACB fusion cancer subtype, which are characterized by the presence of a PRKACB fusion. The patient population and cancer subtype can be associated with or predict the onset of a condition mediated by aberrant PRKACB expression or activity, overexpression of PRKACB, such as, e.g., a cancer or a tumor harboring a PRKACB fusion. The cancer or tumor may be breast cancer. The cancer or tumor may be head and neck squamous cell carcinoma. The methods comprise administering a therapeutic agent, e.g., a PRKACB inhibitor (such as, e.g., a kinase inhibitor or an antibody specific to PRCACB); or a PRKACB fusion inhibitor, i.e., an inhibitor that blocks the activity of the PRKACB fusion but not wild type PRKACB or wild type fusion partner (such as, e.g., an antibody specific to a CEP170:PRKACB or an RBM17:PRKACB fusion protein, e.g., any one of the antibodies described above, a molecule that recognizes the binding partner:PRKACB fusion junction, or an RNA inhibitor that recognizes PRKACB or the fusion junction of a PRKACB fusion, including but not limited to siRNA, dsRNA, shRNA, or any other antisense nucleic acid inhibitor), alone or in combination with e.g., other chemotherapeutic agents or procedures, in an amount sufficient to treat a condition mediated by aberrant PRKACB expression or activity, or overexpression of PRKACB by one or more of the following: impeding growth of a cancer, causing a cancer to shrink by weight or volume, extending the expected survival time of the patient, inhibiting tumor growth, reducing tumor mass, reducing size or number of metastatic lesions, inhibiting the development of new metastatic lesions, prolonging survival, prolonging progression- free survival, prolonging time to progression, and/or enhancing quality of life.

The PRKACB fusion may be inhibited by a PRKACB inhibitor or a PRKACB fusion inhibitorThe therapeutic agent may be a PRKACB inhibitor, such as, e.g., a compound, biological or chemical, which inhibits, directly or indirectly, the expression and/or activity of PRKACB. For example, the PRKACB inhibitors may be an antibody (such as, e.g., antibodies specific to PRKACB) or a small molecule inhibitor (such as, e.g., a kinase inhibitor). The inhibitors may act directly on PRKACB itself, modify the activity of PRKACB, or inhibit the expression of PRKACB. The inhibitors may indirectly inhibit PRKACB activity by inhibiting the activity of proteins or molecules other than PRKACB itself. For example, the inhibitors may modulate the activity of regulatory kinases that phosphorylate or dephosphorylate PRKACB, interfere with binding of ligands, or inhibit the activity of interacting or downstream proteins or molecules.

The PRKACB fusion may be inhibited by a PRKACB fusion inhibitor, such as, e.g., an antibody that recognizes all or part of a PRKACB fusion (such as, e.g., CEP170:PRKACB or RBM17:PRKACB, as described herein) but does not recognize wild type PRKACB or wild type fusion partner (e.g., CEP170 or RBM17). The PRKACB fusion protein (such as, e.g., CEP170:PRKACB or RBM17:PRKACB, as described herein) may be inhibited by an agent that inhibits transcription or translation of the fusion protein, e.g., an RNA inhibitor that recognizes the PRKACB coding sequence, the binding partner (e.g., CEP170 or RBM17), or the binding partner: PRKACB fusion junction, including but not limited to small interfering RNA (siRNA), double stranded RNA (dsRNA), short-hairpin RNA (shRNA), or any other antisense nucleic acid inhibitor. The PRKACB fusion inhibited may be selected from all or a portion of any one of SEQ ID NOs: 1-4.

As used herein, and unless otherwise specified, a "therapeutically effective amount" of a compound is an amount sufficient to provide a therapeutic benefit in the treatment or management of a condition mediated by aberrant PRKACB expression or activity, or overexpression of PRKACB, such as, delaying or minimizing one or more symptoms associated with a cancer or a tumor harboring a PRKACB fusion (such as, e.g., CEP170:PRKACB or RBM17:PRKACB, as described herein). A therapeutically effective amount of a compound means an amount of therapeutic agent, alone or in combination with other therapeutic agents, which provides a therapeutic benefit in the treatment or management of the cancer. The term "therapeutically effective amount" can encompass an amount that improves overall therapy, reduces or avoids symptoms or causes of the condition mediated by aberrant PRKACB expression or activity or overexpression of PRKACB, or enhances the therapeutic efficacy of another therapeutic agent.

The cancer or tumor harboring a PRKACB fusion may be breast cancer. The cancer or tumor harboring a PRKACB fusion may be head and neck squamous cell carcinoma.

The patient to be treated may be suffering from breast cancer and the method for treating the condition may comprise administering to the patient a therapeutically effective amount of a PRKACB inhibitor or a PRKACB fusion inhibitor. The patient to be treated may be suffering from head and neck squamous cell carcinoma, and the method for treating the condition may comprise administering to the patient a therapeutically effective amount of a PRKACB fusion inhibitor or a PRKACB inhibitor.

### Screening Methods (not according to the invention)

Therapeutic agents, such as, e.g., PRKACB inhibitors, and PRKACB fusion inhibitors, used in the therapeutic methods disclosed herein can be evaluated using the screening assays described herein. Also disclosed is a method of identifying an agent useful for treating a condition mediated by aberrant PRKACB expression or activity, or overexpression of PRKACB, such as, e.g., cancer or a tumor harboring a PRKACB fusion, such as e.g., breast cancer or head and neck squamous cell carcinoma, comprising contacting a cell expressing a PRKACB gene fusion or PRKACB fusion protein with a candidate agent and determining whether the expression level of the fusion is decreased or a biological function associated with the fusion is altered. Therapeutic agents can be evaluated in a cell-free system, e.g., a cell lysate or in a reconstituted system. The therapeutic agents may be evaluated in a cell in culture, e.g., a cell expressing a PRKACB fusion (e.g., a mammalian cell, a tumor cell or cell line, a recombinant cell). The therapeutic agents may be evaluated in a cell in vivo (a PRKACB fusion-expressing cell present in a subject, e.g., an animal subject (e.g., an in vivo animal model)).

Exemplary parameters to evaluate in determining the efficacy of a therapeutic agent for treating a condition mediated by aberrant PRKACB expression or activity, or overexpression of PRKACB, such as, e.g., a cancer or a tumor harboring a PRKACB fusion include one or more of:
(i) a change in binding activity, e.g., direct binding of the candidate agent to a PRKACB fusion protein or a binding competition between a known ligand and the candidate agent to a PRKACB fusion protein;
(ii) a change in kinase activity, e.g., phosphorylation levels of a PRKACB fusion protein (e.g., an increased or decreased phosphorylation or autophosphorylation) or a change in phosphorylation of a target of a PRKACB kinase --, a change in kinase activity, e.g., phosphorylation, may be detected by any of western blot (e.g., using an anti- PRKACB antibody or a phosphor-specific antibody, detecting a shift in the molecular weight of a PRKACB fusion protein), mass spectrometry, immunoprecipitation, immunohistochemistry, immunomagnetic beads, among others;
(iii) a change in an activity of a cell containing a PRKACB fusion (e.g., a tumor cell or a recombinant cell), e.g., a change in proliferation, morphology, or tumorigenicity of the cell;
(iv) a change in tumor present in an animal subject, e.g., size, appearance, proliferation, of the tumor;
(v) a change in the level, e.g., expression (transcription and/or translation) level, of a PRKACB fusion protein or nucleic acid molecule; or
(vi) a change in an activity of a signaling pathway involving PRKACB, e.g., phosphorylation or activity of an interacting or downstream target, or expression level of a target gene.

The PRKACB fusion may be a CEP170:PRKACB fusion, or an RBM17:PRKACB fusion.

A change in the activity of a PRKACB fusion, or interaction of a PRKACB fusion with a downstream ligand detected in a cell free assay in the presence of a candidate agent may indicate that the candidate agent will be effective as a therapeutic agent for treatment of a condition mediated by aberrant PRKACB expression or activity, or overexpression of PRKACB, such as, e.g., a cancer or a tumor harboring a PRKACB fusion. The cancer or tumor may be breast cancer. The cancer or tumor may be head and neck squamous cell carcinoma.

A change in an activity of a cell expressing a PRKACB fusion, such as, e.g., CEP170:PRKACB or RBM17:PRKACB, as described herein (e.g., a mammalian cell, a tumor cell or cell line, a recombinant cell) may be detected in a cell in culture. The cell may be a recombinant cell that is modified to express a PRKACB fusion nucleic acid, e.g., is a recombinant cell transfected with a PRKACB fusion nucleic acid. The transfected cell can show a change in response to the expressed PRKACB fusion, e.g., increased proliferation, changes in morphology, increased tumorigenicity, and/or acquired a transformed phenotype. A change in any of the activities of the cell, e.g., the recombinant cell, in the presence of the candidate agent can be detected. For example, a decrease in one or more of: proliferation, tumorigenicity, or transformed morphology, in the presence of the candidate agent can be indicative of an inhibitor of a PRKACB fusion. A change in binding activity or phosphorylation as of PRKACB or its interacting or downstream proteins or molecules described herein may be detected.

A change in a tumor present in an animal subject (e.g., an in vivo animal model) may be detected. A tumor containing animal or a xenograft comprising cells expressing a PRKACB fusion (e.g., tumorigenic cells expressing a PRKACB fusion) may be employed. The therapeutic agents can be administered to the animal subject and a change in the tumor is evaluated. The change in the tumor may include one or more of a tumor growth, tumor size, tumor burden, or survival, is evaluated. A decrease in one or more of tumor growth, tumor size, tumor burden, or an increased survival is indicative that the candidate agent is an inhibitor or modulator.

Also disclosed is a method or assay for screening for agents that modulate (e.g., inhibit) the expression or activity of a PRKACB fusion as described herein. The method includes contacting e.g., a PRKACB fusion, or a cell expressing a PRKACB fusion, with a candidate agent; and detecting a change in a parameter associated with a PRKACB fusion, e.g., a change in the expression or an activity of the PRKACB fusion. The method can, optionally, include comparing the treated parameter to a reference value, e.g., a control sample (e.g., comparing a parameter obtained from a sample with the candidate agent to a parameter obtained from a sample without the candidate agent). If a decrease in expression or activity of the PRKACB fusion is detected, the candidate agent may be identified as an inhibitor. If an increase in expression or activity of the PRKACB fusion is detected, the candidate agent may be identified as an activator. The PRKACB fusion may be a PRKACB gene fusion or PRKACB fusion protein, such as, e.g., a CEP170:PRKACB fusion, or an RBM17:PRKACB fusion.

The contacting step may be detected in a cell-free system, e.g., a cell lysate or in a reconstituted system. The contacting step may be detected in a cell in culture, e.g., a cell expressing a PRKACB fusion (e.g., a mammalian cell, a tumor cell or cell line, a recombinant cell). The contacting step may be detected in a cell in vivo (a PRKACB expressing cell present in a subject, e.g., an animal subject (e.g., an in vivo animal model)).

Exemplary parameters evaluated in identifying an agent that modulates the activity of a PRKACB fusion (e.g., a CEP170:PRKACB fusion, or an RBM17:PRKACB fusion, as disclosed herein) include one or more of:
(i) a change in binding activity, e.g., direct binding of the candidate agent to a PRKACB fusion protein; a binding competition between a known ligand and the candidate agent to a PRKACB fusion protein;
(ii) a change in kinase activity, e.g., phosphorylation levels of a PRKACB fusion protein (e.g., an increased or decreased phosphorylation or autophosphorylation) or a change in phosphorylation of a target of a PRKACB kinase -- a change in kinase activity, e.g., phosphorylation, may be detected by any of western blot (e.g., using an anti- PRKACB antibody or a phosphor- specific antibody, detecting a shift in the molecular weight of a PRKACB fusion protein), mass spectrometry, immunoprecipitation, immunohistochemistry, immunomagnetic beads, among others;
(iii) a change in an activity of a cell containing a PRKACB fusion (e.g., a tumor cell or a recombinant cell), e.g., a change in proliferation, morphology, or tumorigenicity of the cell;
(iv) a change in tumor present in an animal subject, e.g., size, appearance, or proliferation of the tumor;
(v) a change in the level, e.g., expression (transcription or translation) level, of a PRKACB fusion protein or nucleic acid molecule; or
(vi) a change in an activity of a signaling pathway involving PRKACB, e.g., phosphorylation or activity of an interacting or downstream target, or expression level of a target gene.

### Methods for Validating PRKACB Fusions (not according to the invention)

PRKACB fusions, such as, e.g., PRKACB gene fusions (e.g., CEP170:PRKACB gene fusions, or RBM17:PRKACB gene fusions) may be evaluated to ensure that the breakpoints are in-frame and can produce a protein product containing the full kinase domain, i.e., that the breakpoint occurs such that complete triplet codons are intact, and that the RNA sequence will produce a viable protein. The PRKACB gene fusion can be transfected into cells to confirm that the protein is functionally active with respect to kinase activity and oncogenic activity. cDNA encoding the PRKACB fusion protein can be produced by standard solid-phase DNA synthesis. Alternatively the PRKACB fusion cDNA can be produced by RT-PCR using tumor mRNA extracted from samples containing the gene fusion. The DNA amplified can be subcloned into an appropriate vector and characterized by DNA sequence analysis or in vitro/in vivo expression analyses.

Expression vectors containing the PRKACB gene fusion (such as, e.g., a CEP170:PRKACB gene fusion, or an RBM17:PRKACB gene fusion) can be introduced into host cells to thereby produce a PRKACB fusion protein (such as, e.g., a CEP170:PRKACB fusion protein, or an RBM17:PRKACB fusion protein). The PRKACB fusion protein expression vector can be a yeast expression vector, a vector for expression in insect cells, e.g., a baculovirus expression vector, or a vector suitable for expression in mammalian cells. Vector DNA can be introduced into host cells via conventional transformation or transfection techniques. As used herein, the terms "transformation" and "transfection" are intended to refer to a variety of art-recognized techniques for introducing foreign nucleic acid (e.g., DNA) into a host cell.

Cells harboring the expression vector carrying the recombinant PRKACB gene fusion can then be tested for production of the unique fusion protein via standard western blotting using either an antibody probe that detects the gene product itself or that recognizes a tag peptide (e.g., FLAG tag) that can be added to the gene product via the expression vector (using standard, commercially available reagents). Western blotting can be used to confirm the ectopic expression of the encoded PRKACB fusion protein by comparing the samples from cells transfected with the vector containing the PRKACB gene fusion cDNA to cells transfected with the empty expression vector. The functional activity can be assessed by measuring the level of phosphorylation on the kinase or substrate. Comparison of the level of phosphorylation activity between the wild type (normal) form of PRKACB and the PRKACB fusion protein can indicate if the PRKACB fusion protein has elevated activity that could drive oncogenic activity. Whether the PRKACB gene fusion is oncogenic can be assessed by measuring capacity of the expressed PRKACB fusion protein to transform cells, that is, to enable cells to grow and proliferate under conditions which are not permissive for growth of normal cells. One commonly used method of measuring the transforming activity of a kinase is by assessing if expression of the gene product can allow BaF3 cells to grow in the absence of the growth factor IL3, which is required for the survival and growth of BaF3 cells. Another assay for measuring transforming activity is a soft agar growth assay. This is another standard method which tests the capacity of an introduced gene product to confer the ability to grow in a soft agar matrix, or anchorage-independent conditions. These methods and others can be used to test the oncogenic activity of a PRKACB gene fusion (such as, e.g., a CEP170:PRKACB gene fusion, or an RBM17:PRKACB gene fusion) and provide a level of validation of a PRKACB fusion protein (such as, e.g., a CEP170:PRKACB fusion protein, or an RBM17:PRKACB fusion protein) as a potential target for treating patients that harbor these fusions.

A change in an activity of a cell can be detected in a cell in culture, e.g., a cell expressing a fusion (e.g., a mammalian cell, a tumor cell or cell line, a recombinant cell). The transfected cell can show a change in response to the expressed fusion, e.g., increased proliferation, changes in morphology, increased tumorigenicity, and/or an acquired transformed phenotype.

To further validate the biological implication of the gene fusion, a change in any of the activities of the cell, e.g., the recombinant cell, in the presence of a known inhibitor of one of the fusion partners, e.g., a PRKACB inhibitor, can be detected. For example, a decrease in one or more of: proliferation, tumorigenicity, and transformed morphology, in the presence of the PRKACB inhibitor can be indicative of an inhibitor of a fusion. A change in binding activity or phosphorylation of PRKACB or its interacting or downstream proteins or molecules may be detected.

Unless otherwise required by context, singular terms shall include the plural and plural terms shall include the singular. The use of "or" means "and/or" unless stated otherwise. The use of the term "including," as well as other forms, such as "includes" and "included," is not limiting. All ranges given in the application encompass the endpoints unless stated otherwise.

Those skilled in the art will recognize, or be able to ascertain using no more than routine experimentation, many equivalents to the specific embodiments of the invention described herein. Such equivalents are intended to be encompassed by the following claims.

## Claims

1. A method of detecting a PRKACB gene fusion in a biological sample, wherein the PRKACB gene fusion is a CEP170:PRKACB or RBM17:PRKACB fusion, said method comprising:
a) contacting a biological sample from a mammal with a reagent that is an oligonucleotide that hybridizes to a fusion junction of the PRKACB gene fusion of SEQ ID NO: 1 or 3; and
b) detecting binding between the PRKACB gene fusion and the reagent.

2. The method of claim 1 wherein the biological sample is from a human cancer.

3. The method of claim 2, wherein the human cancer is breast cancer.

4. The method of claim 2, wherein the human cancer is head and neck squamous cell carcinoma.

5. The method of any of claims 1 to 4, wherein the reagent of steps a) and b) hybridizes to:
(a) nucleotides 1108-1110, 1107-1111, 1104-1113, 1099-1118, 1084-1133, 1059-1158, 1034-1184, or 1009-1208 of SEQ ID NO: 1; or
(b) nucleotides 505-507, 504-508, 501-510, 496-515, 481-530, 456-555, 431-580, or 406-605 of SEQ ID NO: 3.

6. The method of claim 5, wherein the reagent hybridizes to nucleotides 1104-1113 of SEQ ID NO:1.

7. The method of claim 5, wherein the reagent hybridizes to nucleotides 501-510 of SEQ ID NO:3.

## Patentansprüche

1. Verfahren zum Detektieren einer PRKACB-Genfusion in einer biologischen Probe, wobei die PRKACB-Genfusion eine CEP170:PRKACB- oder RBM17:PRKACB-Fusion ist, das Verfahren umfassend:
a) Inkontaktbringen einer biologischen Probe von einem Säugetier mit einem Reagenz, das ein Oligonukleotid ist, das mit einer Fusionsverbindung der PRKACB-Genfusion von SEQ ID NO: 1 oder 3 hybridisiert; und
b) Detektieren einer Bindung zwischen der PRKACB-Genfusion und dem Reagenz.

2. Verfahren nach Anspruch 1, wobei die biologische Probe von einem menschlichen Krebs ist.

3. Verfahren nach Anspruch 2, wobei der menschliche Krebs Brustkrebs ist.

4. Verfahren nach Anspruch 2, wobei der menschliche Krebs ein Kopf- und Hals-Plattenepithelkarzinom ist.

5. Verfahren nach einem der Ansprüche 1 bis 4, wobei das Reagenz aus Schritten a) und b) mit Folgendem hybridisiert:
(a) Nukleotiden 1108-1110, 1107-1111, 1104-1113, 1099-1118, 1084-1133, 1059-1158, 1034-1184 oder 1009-1208 von SEQ ID NO: 1; oder
(b) Nukleotiden 505-507, 504-508, 501-510, 496-515, 481-530, 456-555, 431-580, oder 406-605 von SEQ ID NO: 3.

6. Verfahren nach Anspruch 5, wobei das Reagenz mit Nukleotiden 1104-1113 von SEQ ID NO: 1 hybridisiert.

7. Verfahren nach Anspruch 5, wobei das Reagenz mit Nukleotiden 501-510 von SEQ ID NO: 3 hybridisiert.

## Revendications

1. Procédé de détection d'une fusion du gène PRKACB dans un échantillon biologique, dans lequel la fusion du gène PRKACB est une fusion CEP170:PRKACB ou RBM17:PRKACB, ledit procédé comprenant :
a) la mise en contact d'un échantillon biologique provenant d'un mammifère avec un réactif qui est un oligonucléotide qui s'hybride à une jonction de fusion de la fusion du gène PRKACB de SEQ ID NO : 1 ou 3 ; et
b) la détection de la liaison entre la fusion du gène PRKACB et le réactif.

2. Procédé selon la revendication 1, dans lequel l'échantillon biologique provient d'un cancer humain.

3. Procédé selon la revendication 2, dans lequel le cancer humain est le cancer du sein.

4. Procédé selon la revendication 2, dans lequel le cancer humain est un carcinome épidermoïde de la tête et du cou.

5. Procédé selon l'une quelconque des revendications 1 à 4, dans lequel le réactif des étapes a) et b) s'hybride aux :
(a) nucléotides 1108 à 1110, 1107 à 1111, 1104 à 1113, 1099 à 1118, 1084 à 1133, 1059 à 1158, 1034 à 1184 ou 1009 à 1208 de SEQ ID NO : 1 ; ou
(b) nucléotides 505 à 507, 504 à 508, 501 à 510, 496 à 515, 481 à 530, 456 à 555, 431 à 580 ou 406 à 605 de SEQ ID NO : 3.

6. Procédé selon la revendication 5, dans lequel le réactif s'hybride aux nucléotides 1104 à 1113 de SEQ ID NO : 1.

7. Procédé selon la revendication 5, dans lequel le réactif s'hybride aux nucléotides 501 à 510 de SEQ ID NO : 3.
